# EUROPÄISCHE PATENTSCHRIFT

(11) **EP 3 126 430 B1**
(45) Veröffentlichungstag und Bekanntmachung des Hinweises auf die Patenterteilung: **11.12.2019**
(21) Anmeldenummer: 15718153.8
(22) Anmeldetag: 02.04.2015
(51) Int. Cl.: C08G 71/00, C07D 317/36, C08G 71/04, C08L 75/12

(54) **ZWEI-KOMPONENTEN-BINDEMITTEL MIT CYCLOCARBONAT- UND EPOXIDGRUPPEN**
TWO-COMPONENT BINDER HAVING CYCLOCARBONATE AND EPOXIDE GROUPS
LIANT À DEUX COMPOSANTS COMPORTANT DES GROUPS CARBONATE CYCLIQUE ET ÉPOXYDE

(30) Priorität: 04.04.2014 DE 102014206574
(43) Veröffentlichungstag der Anmeldung: 08.02.2017
(73) Patentinhaber: Henkel AG & Co. KGaA, 40589 Düsseldorf (DE)
(72) Erfinder: LAMMERSCHOP, Olaf, 47802 Krefeld (DE); KINZELMANN, Hans-Georg, 50259 Pulheim (DE); HEMERY, Therese, E-08007 Barcelona (ES)
(86) Internationale Anmeldenummer: PCT/EP2015/057367
(87) Internationale Veröffentlichungsnummer: WO 2015/150543

(56) Entgegenhaltungen:
- DE-A1-102004 035 542
- US-B1- 6 407 198
- US-B2- 6 627 761

## Beschreibung

Die vorliegende Erfindung betrifft ein Bindemittel-System, das eine Harzkomponente und eine Härterkomponente enthält, wobei das Harz mindestens eine mindestens zwei zyklische Carbonatgruppen tragende Verbindung und mindestens eine mindestens zwei Epoxidgruppen tragende Verbindung umfasst, und der Härter mindestens ein multifunktionelles Amin umfasst, sowie die Verwendung des Bindemittel-Systems als Kleb-/Dichtstoff und die Verwendung dieses Kleb-/Dichtstoffes.

Zwei-Komponenten-Bindemittel-Systeme, insbesondere auf Basis von Polyolen und NCO-terminierten Verbindungen, sind seit langem Stand der Technik. Sie werden beispielsweise im Bereich der metallverarbeitenden Industrie, der Fahrzeugindustrie, der Elektroindustrie, der Verpackungsindustrie oder der Bauindustrie als Kleb-, Dicht-, Füllstoffe oder Vergußmassen (Casting) eingesetzt. Nachteilig an den als sogenanntes Harz eingesetzten NCO-Gruppen tragenden Polyurethanen ist die Empfindlichkeit gegenüber Feuchtigkeit. Bei der Lagerung dieser Verbindungen müssen daher entsprechend dichte Gebinde zum Einsatz kommen. Einmal geöffnete Gebinde sind in der Regel sofort bzw. kurzfristig zu verbrauchen, um einen Qualitätsverlust zu vermeiden. Auch muss in der Regel die Polyolkomponente vor dem Mischen mit der Harzkomponente sorgfältig getrocknet werden, da ansonsten eine Restmenge an Feuchtigkeit zu unerwünschter Blasenbildung im Klebstofffilm führen kann, die unter Umständen Nachteile bei der Endanwendung verursacht. Ein weiterer Nachteil zumindest einiger Bindemittel-Systeme auf Basis von Zwei-Komponenten-Polyurethan-Klebstoffen ist die Toxikologie von monomeren Isocyanaten, insbesondere leicht flüchtigen und/oder leicht migrierenden monomeren Diisocyanaten, in der Harzkomponente. Die Anwendung von Produkten mit einem hohen Gehalt an leichtflüchtigen Diisocyanaten erfordert seitens des Anwenders aufwendige Arbeitsschutzmaßnahmen, insbesondere aufwendige Maßnahmen zur Reinhaltung der Atemluft, gesetzlich vorgegeben durch die höchstzulässige Konzentration von Arbeitsstoffen als Gas, Dampf oder Schwebstoff in der Luft am Arbeitsplatz (jährlich aktualisierte MAK-Wert-Liste der Technischen Regel TRGS 900 des Bundesministeriums für Arbeit und Soziales).

US 6627761 beschreibt ein Verfahren zur Herstellung von multifunktionelle carbonat-Harzen. DE 102004035542 A1 offenbart ein zwei-komponenten Bindemittel-System, welches die Komponenten (A) und (B) enthält, wobei Komponent (A) eine mindestens zwei zyklische Carbonatgruppen tragende Verbindung oder ein Gemisch aus zwei oder mehr mindestens zwei zyklische Carbonatgruppen tragende Verbindungen ist und Komponent (B) eine mindestens zwei (-NHR-)-Atomgruppierungen tragende Verbindung oder ein Gemisch aus zwei oder mehr mindestens zwei (-NHR-)-Atomgruppierungen tragende Verbindungen ist, mit R = H, Alkyl- oder Aryl-Rest. US 4607198 offenbart chemisch resistente Materialen mit guten mechanischen Eigenschaften basierend auf Polycyclocarbonaten, welche durch eine Reaktion von Aminen mit epoxy-Endgruppen enthaltend Oligocyclocarbonaten hergestellt werden. Freie monomere Polyisocyanate können aber auch in die Beschichtung oder Verklebung, oder zum Teil auch in die beschichteten oder verklebten Materialien hinein "wandern". Solche wandernden Bestandteile werden in Fachkreisen häufig als "Migrate" bezeichnet. Durch Kontakt mit

Feuchtigkeit werden die Isocyanatgruppen der Migrate kontinuierlich zu Aminogruppen umgesetzt.

Im Verpackungsbereich, speziell bei Lebensmittelverpackungen, sind Migrate besonders unerwünscht. Einerseits kann die Wanderung der Migrate durch das Verpackungsmaterial hindurch zu einer Kontamination des verpackten Gutes führen, andererseits sind, abhängig von der Menge des migrierbaren freien monomeren Polyisocyanates, lange Wartezeiten notwendig, bevor das Verpackungsmaterial "Migrat-frei" ist und verwendet werden darf.

Ein weiterer unerwünschter Effekt, der durch die Migration monomerer Polyisocyanate hervorgerufen werden kann, ist der sogenannte Antisiegeleffekt bei der Herstellung von Beuteln oder Tragetaschen aus kaschierten Kunststoff-Folien: Häufig enthalten die kaschierten Kunststoff- Folien Gleitmittel auf Basis von Fettsäureamiden. Durch Reaktion von migriertem monomeren Polyisocyanat mit dem Fettsäureamid und Feuchtigkeit werden an der Folienoberfläche Harnstoffverbindungen gebildet, die einen Schmelzpunkt besitzen, der über der Versiegelungstemperatur der Kunststoff-Folien liegen kann. Dadurch entsteht eine artfremde Antisiegel-Schicht zwischen den zu versiegelnden Folienteilen, die einer einheitlichen Siegel-Nahtbildung entgegenwirkt.

Produkte auf Basis von zyklischen Carbonatgruppen tragenden Verbindungen und aliphatischen Polyaminen sind prinzipiell bekannt, beispielsweise aus der WO 2006/010408. Obwohl die darin beschriebenen Produkte die oben erwähnten Nachteile überwinden und gleichzeitig gute Klebe/Dichteigenschaften aufweisen, erfordern sie hohe Härtungstemperaturen von mindestens 80°C, durchschnittlich eher um 100°C, was für bestimmte Anwendungsgebiete nachteilig oder sogar unpraktikabel ist. Bei niedrigeren Temperaturen, wie beispielsweise 40°C, ist allerdings die Härtung nicht vollständig und später die Haftung zu gering.

Aufgabe der vorliegenden Erfindung war es daher, ein Bindemittel-System zur Verfügung zu stellen, das diese Nachteile überwindet und schon bei niedrigen Temperaturen eine ausreichende Härtung ermöglicht und gute Hafteigenschaften zeigt.

Die Erfinder haben überraschenderweise gefunden, dass sich diese Aufgabe durch ein Bindemittelsystem lösen lässt, dass zusätzlich zu einer zyklische Carbonatgruppen tragenden Harzkomponente eine Epoxidharzkomponente verwendet. Die derart erhaltenen Bindemittelsysteme zeigen bereits bei Temperaturen um 40°C eine vollständige Härtung bei mit bekannten Systemen vergleichbaren Hafteigenschaften.

In einem ersten Aspekt betrifft die Erfindung daher ein Bindemittel-System das die Komponenten (A) und (B) enthält, wobei Komponente (A) mindestens eine mindestens zwei zyklische Carbonatgruppen tragende Verbindung und mindestens eine mindestens zwei Epoxidgruppen tragende Verbindung umfasst; und Komponente (B) mindestens eine mindestens zwei (-NHR-)-Atomgruppierungen tragende Verbindung umfasst, wobei R H, ein Alkyl- oder Aryl-Rest ist. Der Alkylrest kann geradkettig oder verzweigt sein, gesättigt oder ein- oder mehrfach ungesättigt, substituiert oder unsubstituiert und hat insbesondere 1 bis 30 Kohlenstoffatome. Der Arylrest kann monozyklisch oder polyzyklisch sein, substituiert oder unsubstituiert und hat insbesondere 6 bis 22 Kohlenstoffatome.

In einem weiteren Aspekt betrifft die Erfindung Verfahren zur Herstellung eines Kleb-/Dichtstoffes unter Verwendung des hierin beschriebenen Bindemittel-Systems. In diesen Verfahren wird die Komponente (A) mit der Komponente (B) im Verhältnis der Carbonatgruppen zu primären Aminogruppen von 30:1 bis 0,2:1, bevorzugt 10:1 bis 0,4:1, weiter bevorzugt von 5:1 bis 0,5:1, besonders bevorzugt von 2:1 bis 0,6:1, insbesondere bevorzugt 1,1:1 bis 0,9:1 und am meisten bevorzugt ungefähr 1:1 gemischt. In Abwesenheit von primären Aminogruppen ist das Mischungsverhältnis auf sekundäre Aminogruppen anzuwenden.

In noch einem weiteren Aspekt betrifft die Erfindung die Verwendung des hierin beschriebenen Bindemittel-Systems als Zweikomponenten-Klebstoff zum Verkleben von Papier, Pappe, Holz, Kunststoff, Metall oder Steingut, insbesondere als lösemittelfreier oder lösemittelhaltiger Kaschierklebstoff oder zur Herstellung von Vergussmassen.

Schließlich erfasst die Erfindung auch Verfahren zur Herstellung von Folienverbunden, wobei mindestens zwei gleiche oder unterschiedliche Kunststofffolien unter Verwendung eines hierin beschriebenen Bindemittel-Systems teil- oder vollflächig verklebt werden, sowie den derart hergestellten Folienverbund.

Die im vorliegenden Text angegebenen Molekulargewichte beziehen sich, soweit nicht anders angegeben, auf das Gewichtsmittel des Molekulargewichts (Mw). Alle Molekulargewichtsangaben beziehen sich, soweit nicht anders angegeben ist, auf Werte, wie sie durch Gelpermeationschromatographie (GPC) nach der Norm DIN 55672-1:2007-08 erhältlich sind.

"Mindestens ein", wie hierin verwendet, bedeutet 1 oder mehr, d.h. 1, 2, 3, 4, 5, 6, 7, 8, 9 oder mehr. Bezogen auf einen Inhaltsstoff bezieht sich die Angabe auf die Art des Inhaltsstoffs und nicht auf die absolute Zahl der Moleküle. "Mindestens ein Polyol" bedeutet somit beispielsweise mindestens eine Art von Polyol, d.h. dass eine Art von Polyol oder eine Mischung mehrerer verschiedener Polyole verwendet werden kann. Zusammen mit Gewichtsangaben bezieht sich die Angabe auf alle Verbindungen der angegebenen Art, die in der Zusammensetzung/Mischung enthalten sind, d.h. dass die Zusammensetzung über die angegebene Menge der entsprechenden Verbindungen hinaus keine weiteren Verbindungen dieser Art enthält.

Alle Prozentangaben, die im Zusammenhang mit den hierin beschriebenen Zusammensetzungen gemacht werden, beziehen sich, sofern nicht explizit anders angegeben auf Gew.-%, jeweils bezogen auf die betreffende Mischung.

"Ungefähr" oder "ca.", wie hierin im Zusammenhang mit einem Zahlenwert verwendet bezieht sich auf den Zahlenwert ±10 %, vorzugsweise ±5%.

Überraschend wurde gefunden, dass die hierin beschriebenen Bindemittel-Systeme eine im Wesentlichen vollständige Umsetzung bereits bei vergleichsweise niedrigen Temperaturen aufweisen, sich als Kleb/Dichtstoffe eignen und sich durch eine gute Adhäsion auf Oberflächen unterschiedlichster Materialien auszeichnen. Die im wesentlichen NCO-Gruppen freien Polyurethan-Kleb-/Dichtstoffe können dabei in Substanz oder als Lösung in gebräuchlichen organischen Lösemitteln verwendet werden. Im wesentlichen frei an NCO-Gruppen bedeutet, dass der NCO-Gehalt in Komponente (A) < 0,1 Gew.- % beträgt (bestimmt nach Spiegelberger, EN ISO 11909:2007-05).

Als die mindestens eine mindestens zwei zyklische Carbonatgruppen tragende Verbindung können beliebige Polymere eingesetzt werden, sofern sie keine anderen funktionellen Gruppen aufweisen, welche die Reaktion mit der Komponente (B) stören könnten. Die mindestens eine mindestens zwei zyklische Carbonatgruppen tragende Verbindung kann sowohl linear als auch verzweigt sein. Wie bereits oben erwähnt, bedeutet der Ausdruck "mindestens eine" in diesem Zusammenhang, dass das Bindemittel-System eine oder mehrere Verbindungen enthalten kann, die jeweils mindestens zwei zyklische Carbonatgruppen tragen. "Mindestens zwei zyklische Carbonatgruppen" bedeutet, dass die Verbindungen zwei oder mehr, vorzugsweise aber genau zwei, insbesondere terminale, zyklische Carbonatgruppen tragen.

Die zyklischen Carbonatgruppen sind vorzugsweise an den Polymerkettenenden angeordnet, jedoch können in manchen Fällen auch Verbindungen eingesetzt werden, welche diese Gruppen in statistischer Verteilung über die gesamte Polymerkette enthalten. Die zyklischen Carbonatgruppen können somit sowohl in die Hauptkette eingebaut als auch seitenständig angeordnet sein.

Bevorzugt ist die Verbindung, die mindestens zwei zyklische Carbonatgruppen trägt, ein Polymer, wobei das Polymer ausgewählt ist aus der Gruppe der fettchemischen Verbindungen, Polyether, Polyetherpolyole, Polyester, Polyesterpolyole, Polycarbonate, Polycarbonsäuren, Polyacrylate, Polymethacrylate, Polyamide, Polyamine, Polyurethane oder Mischungen daraus. Als fettchemische Verbindungen werden bevorzugt Rizinusöl oder Dimerdiol eingesetzt, die noch alkoxyliert sind. Im Rahmen dieser Erfindung besonders bevorzugt sind Polyurethane.

Unter Polyamiden werden solche verstanden, die keine aminischen NH-Gruppen aufweisen.

Unter Cyclocarbonaten, die auch als zyklische oder cyclische Carbonate bezeichnet werden, sind Strukturen zu verstehen, bei denen eine Kohlensäureestergruppe Teil einer Ringstruktur ist gemäß Formel (I): 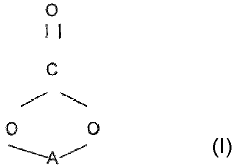 wobei
A (C(R¹R²))ₙ ist, mit n > 2, bevorzugt n = 2 oder 3, insbesondere bevorzugt n = 2;
R¹, R² jeweils unabhängig ausgewählt werden aus Wasserstoff, einem gesättigten oder ungesättigten, gerad- oder verzweigtkettigen oder zyklischen, aromatischen oder arylaliphatischen, gegebenenfalls substituierten, Kohlenwasserstoffrest mit 1 bis 12 Kohlenstoffatomen, einem Etherrest mit 1 bis 12 Kohlenstoffatomen und bis zu 3 Sauerstoffatomen, R³X, wobei R³ einen zweiwertigen aliphatischen, cycloaliphatischen, aromatischen, arylaliphatischen oder etherhaltigen ggf. substituierten Kohlenwasserstoffrest mit 1 bis 20 Kohlenstoffatomen und X eine Hydroxy-, Epoxy-, Carbonsäure- oder Carbonsäureestergruppe darstellt, oder Z, wobei Z eine ungesättigte polymerisierbare Gruppe darstellt, insbesondere eine Vinyl-, (Meth)Acryl-, Maleinsäure-, Fumarsäure-, Itaconsäure- oder Crotonsäureestergruppe.

Konkrete Beispiele für Cyclocarbonate sind, ohne darauf beschränkt zu sein, Ethylencarbonat (1,3-Dioxolan-2-on); Propylencarbonat (4-Methyl-1,3-dioxolan-2-on); Glycerincarbonat (4-Methylhydroxy-1,3-dioxolan-2-on); 5-Ethyl-5-(Hydroxymethyl)-1,3-Dioxan-2-on; 1,3-Dioxan-2-on; 5-((Allyloxy)methyl)-5-ethyl-1,3-dioxan-2-on; oder 1,3-Dioxepin-2-on. Epoxidgruppen-tragende cyclische Carbonate werden beispielsweise in der DE 3726497 A1 beschrieben.

Zyklische Carbonate werden beispielsweise durch Umesterung von Kohlensäureestern, wie z.B. Dimethylcarbonat, Diethylcarbonat, Diphenylcarbonat, Ethylencarbonat oder Propylencarbonat mit Polyolen erhalten, wobei die Polyole bevorzugt mindestens drei Hydroxylgruppen tragen, von denen je zwei mit Kohlensäureestern in einer Umesterung zu cyclischen Fünfring- oder Sechsring-Carbonaten reagieren. Als mehrwertige Polyole sind beispielsweise zu nennen: Glycerin, Diglycerin, Triglycerin, Polyglycerin, Zuckeralkohole (z.B. Xylit, Mannit, Erytherit), Di- und Trimethylolpropan, Di- und Trimethylolethan, Pentaerythrit, Dipentaerythrit. Gylcerin ist hierbei besonders bevorzugt. Die Herstellung der cyclischen Carbonate aus den Polyolen geschieht in dem Fachmann geläufiger Weise, insbesondere durch Umsetzung der Polyole mit den Carbonaten im stöchiometrischen Verhältnis 1,0:1,0 bis 1,0:10,0 (Verhältnis von 1,2- oder 1,3- Glykolgruppen zu Carbonatgruppen), insbesondere unter Katalyse. Als Katalysatoren kommen z.B. basische Katalysatoren, wie zum Beispiel: Carbonate, Bicarbonate, Alkoholate, Carboxylate, Hydroxide oder Oxide der Alkali- und Erdalkalimetalle in Frage, wie auch Lewis-saure Substanzen, wie zum Beispiel organische Verbindungen des zwei- oder vierwertigen Zinns oder Titans, zum Beispiel Zinn-(II)-octoat, Zinn-(II)-laureat, Dibutylzinnoxid oder Titantetrabutylat. Die Katalysatoren können z.B. in einer Menge von 0,01 bis 1,0 Gew.-% bezogen auf Polyol und Kohlensäureester zugesetzt werden.

Zyklische Carbonate können auch durch Umsetzung von Kohlendioxid mit Epoxidverbindungen nach bekannter Art und Weise erhalten werden. Derartige Umsetzungen sind beispielsweise in WO 84/03701, DE-A 3529263 oder DE-A 3600602 beschrieben.

Durch Reaktion von Polyolen mit Phosgen sind sowohl aliphatische als auch aromatische cyclische Carbonate erhältlich (z. B. US 3,624,016).

Die Ausstattung des Polymeren mit mindestens zwei zyklischen Carbonatgruppen, im Folgenden als Funktionalisierung bezeichnet, kann während des Polymerkettenaufbaus erfolgen, wobei entsprechende monomere Bausteine, die Cyclocarbonatgruppen enthalten, eingesetzt werden. Bevorzugt ist aber die nachträgliche Funktionalisierung eines bereits hergestellten Polymeren. Besonders bevorzugt ist hierbei die Addition von zyklischen Hydroxyalkylcarbonaten an Anhydridgruppen oder Isocyanatgruppen tragende Polymere. Prinzipiell ist ein entsprechendes Verfahren ist in der EP 0328150 A2 beschrieben. Bevorzugt werden für die Additionsreaktionen zyklische Hydroxyalkylcarbonate mit 5-gliedrigen oder 6-gliedrigen Carbonatringen eingesetzt. Ganz besonders bevorzugt wird Glycerincarbonat verwendet. Ebenso ist es möglich solche zyklischen Hydroxyalkylcarbonate durch Umesterung einzureagieren. Dabei können beispielsweise C1-C4-Alkylestergruppen des Polymeren direkt umgesetzt werden, oder es ist möglich Hydroxygruppen der Polymere mit einer Estergruppe eines niedermolekularen C-2 bis C6-Dicarbonsäureesters umzusetzen und anschließend die verbleibenden C1-C4-Alkyl-Estergruppen mit einem Hydroxyalkylcarbonat umzusetzen. Unter niedermolekularen Dicarbonsäureestern sind solche zu verstehen, deren Dicarbonsäurerest aus 2 bis 44 C-Atomen, bevorzugt 2 bis 12 C-Atomen, besonders bevorzugt 2 bis 6 C-Atomen aufgebaut ist und eine lineare oder verzweigte aliphatische, cycloaliphatische oder aromatische Struktur aufweisen kann. Eine weitere Möglichkeit Hydroxyalkylcarbonate einreagieren zu lassen ist deren Reaktion mit Säurehalogeniden, insbesondere Carbonsäurehalogeniden.

In einer weiteren Ausführungsform der Erfindung werden als Verbindungen, die mindestens zwei zyklische Carbonatgruppen tragen, solche eingesetzt, die durch Addition von Kohlendioxid an Epoxidgruppen-haltige Polymere erhältlich sind. Prinzipiell ist ein derartiges Additionsverfahren in DE-OS 3529263 bzw. DE-OS 3600602 beschrieben.

Durch die Auswahl der Basisverbindung und die Auswahl der Funktionalisierung mit den zyklischen Carbonatgruppen ist es möglich Polymere zu erhalten, die Urethangruppen enthalten oder nur Estergruppen. Dadurch kann die Viskosität des Polymeren beeinflusst werden.

Bevorzugt sind Polyurethane, die mit mindestens zwei zyklischen Carbonatgruppen funktionalisiert sind. Die zyklischen Carbonatgruppen sind dabei insbesondere an den Termini angeordnet. Derartig funktionalisierte Polymere werden in einer zweistufigen Synthese hergestellt. In einem ersten Schritt wird ein Polyol oder eine Polyolmischung mit einem stöchiometrischen Überschuss von Polyisocyanat umgesetzt, um ein NCO-terminiertes Polyurethan-Präpolymer zu erhalten. Dieses wird dann in einem zweiten Schritt mit den zyklischen Carbonatgruppen funktionalisiert, beispielsweise durch Umsetzen von zyklischem Hydroxyalkylcarbonat, insbesondere einem, das einen 5-gliedrigen oder 6-gliedrigen Carbonatring aufweist, ganz besonders bevorzugt Glycerincarbonat, mit den terminalen Isocyanatgruppen.

Es ist daher bevorzugt, dass die Verbindung, die mindestens zwei zyklische Carbonatgruppen trägt, das Umsetzungsprodukt von einem Isocyanatgruppen-tragenden Polymer, insbesondere einem Isocyanatgruppen-terminierten Polyurethanpräpolymer, mit einem Hydroxyalkylcarbonat, insbesondere einem, das einen 5-gliedrigen oder 6-gliedrigen Carbonatring aufweist, ganz besonders bevorzugt Glycerincarbonat, ist.

Die in der Herstellung des Polymers eingesetzten Polyole können alle üblicherweise für die Polyurethansynthese eingesetzten Polyole, beispielsweise Polyesterpolyole oder Polyetherpolyole, insbesondere Polyetherpolyole, wie Polypropylenglykol oder Polyethylenglykol, sein. Die eingesetzten Polyole haben vorzugsweise ein mittleres Molekulargewicht (Mw) von 60 bis 4.000 g/mol, vorzugsweise 75 bis 2.000 g/mol.

Die eingesetzten Polyisocyanate sind insbesondere Diisocyanate, besonders bevorzugt aromatische Diisocyanate. Beispiele für geeignete Diisocyanate sind Methylendiphenyldiisocyanate (MDI), wie 4,4'-Methylendiphenyldiisocyanat, 2,4'-Methylendiphenyldiisocyanat oder 2,2'-Methylendiphenyldiisocyanat, sowie ihren Mischungen.

Die mindestens eine mindestens zwei zyklische Carbonatgruppen tragende Verbindung ist daher vorzugsweise ein Cyclocarbonat-terminiertes Polyurethanpräpolymer aus einem Polyetherpolyol und einem aromatischen Diisocyanat, wie MDI.

Das Molekulargewicht (Mw) der mindestens einen mindestens zwei zyklische Carbonatgruppen tragenden Verbindung liegt vorzugsweise von 1.500 g/mol bis 100.000 g/mol, insbesondere bevorzugt von 2.000 g/mol bis 50.000 g/mol.

In einer weiteren Ausführungsform kann in dem Bindemittel-System auch eine weitere niedermolekulare Verbindung enthalten sein, die zyklische Carbonatgruppen aufweist. Diese Komponente sollte ein Molekulargewicht (Mw) < 1.000 g/mol aufweisen, bevorzugt < 800 g/mol, und mindestens zwei zyklische Carbonatgruppen enthalten. Es kann sich dabei beispielsweise um mit CO₂ umgesetzte Di-Epoxide handeln oder um Di- oder Tricarbonsäureester, die an den Estergruppen mit den oben erwähnten hydroxyfunktionellen zyklischen Carbonaten umgesetzt wurden. Komponenten dieser Art werden auch als Reaktivverdünner bezeichnet und können beispielsweise in Mengen bis zu 60 Gew.-%, bevorzugt bis zu 25 Gew.% bezogen auf (A) zur Beeinflussung der Viskosität des Bindemittel-Systems zugesetzt werden.

Die Komponente (A) enthält des Weiteren mindestens eine, mindestens zwei Epoxidgruppen tragende Verbindung. Die hierin verwendbaren Verbindungen sind vorzugsweise Epoxidharze, wie zum Beispiel Polyglycidylepoxid-Verbindungen oder Epoxid-Novolake. Geeignete Polyglycidylepoxide schließen ein, ohne darauf beschränkt zu sein, Polyglycidylether, Poly(β-methylglycidyl)ether, Polyglycidylester und Poly(β-methylglycidyl)ester und Mischungen der vorgenannten Epoxidharze. Geeignete Polyglycidylether oder -ester sind beispielsweise Diglycidylether oder -ester von aliphatischen Diolen oder Dicarbonsäuren. Ebenfalls geeignet sind cycloaliphatische Epoxidharze wie beispielsweise (Di-)Ether oder (Di-)Ester auf Basis von 3,4-Epoxycyclohexyl-methanol.

Das mindestens eine Epoxidharz wird in verschiedenen Ausführungsformen ausgewählt aus Diglycidylethern basierend auf Propylenglykol oder Ethylenglykol.

Es ist allgemein bevorzugt, dass die mindestens zwei Epoxidgruppen tragende Verbindung ein aliphatisches Epoxid ist. Bevorzugt sind solche Epoxide, die ein Epoxid-Äquivalentgewicht (EEW, epoxy equivalent weight) von 100 bis 500 g/mol, vorzugsweise 120-350 g/mol, aufweisen. Das EEW bezeichnet dabei die Masse an Epoxidverbindung, die 1 mol Epoxidgruppen enthält. Sie ist gemäß der Norm DIN EN ISO 3001:1999-11 bestimmbar.

Die Menge des Epoxids in Komponente (A) beträgt vorzugsweise 5 bis 30 Gew.-% bezogen auf die Gesamtfeststoffmenge (Cyclocarbonat-Verbindung und Epoxid). Dementsprechend ist die Cyclocarbonatverbindung in einer Menge von 70 bis 95 Gew.-% bezogen auf die Gesamtfeststoffmenge enthalten.

In dem erfindungsgemässen Bindemittel-System, ist neben der Komponente (A) mindestens ein multifunktionelles Amin, d.h. mindestens eine mindestens zwei (-NHR-)-Atomgruppierungen tragende Verbindung oder ein Gemisch aus zwei oder mehr mindestens zwei (-NHR-)-Atomgruppierungen tragende Verbindungen als Komponente (B) enthalten, mit R = H, Alkyl- oder Aryl-Rest.

Die Verbindungen der Komponente (B) können sowohl linear als auch verzweigt sein. Das Molekülgerüst von Komponente (B) kann aliphatische, aromatische, aliphatisch-aromatische, cycloaliphatische und heterocyclische Strukturen enthalten. Im Molekül können primäre und/oder sekundäre und tertiäre Amine vorhanden sein, jedoch müssen mindestens zwei (-NHR-)-Atomgruppierungen, bevorzugt zwei Aminogruppen, enthalten sein. Die Aminfunktionen selbst sind aliphatisch, das heißt, dass die dem Aminstickstoff unmittelbar benachbarten Kohlenstoffatome nicht Teil einer aromatischen Ringstruktur sind.

Die Komponente (B) enthält vorzugsweise ein multifunktionelles Amin als Komponente (B1), insbesondere mit einem mittleren Molekulargewicht (Mw) von 60 g/mol bis 500 g/mol, bevorzugt von 60 g/mol bis 300 g/mol, und/oder ein multifunktionelles Amin als Komponente (B2), insbesondere mit einem mittleren Molekulargewicht (Mw) von >500 g/mol. Besonders bevorzugt sind Mischungen von (B1) und (B2). Das Gewichtsverhältnis von (B1) zu (B2) in eingesetzten Mischungen von (B1) mit (B2) beträgt 0,5:20 bis 20:0,5. Die Obergrenze des Molekulargewichtes (Mw) von Komponente (B2) liegt bei ca. 5.000.000 g/mol. Bevorzugt weist Komponente (B2) ein mittleres Molekulargewicht (Mw) von 600 g/mol bis 20.000 g/mol, insbesondere bevorzugt von 800 g/mol bis 2.000 g/mol auf.

Die Komponente (B1) wird als Einzelkomponente oder auch als Mischung der entsprechenden, als Komponente (B1) einsetzbaren Verbindungen eingesetzt. Komponente (B1) wird bevorzugt aus der Gruppe der Alkylendiamine und/oder Cycloalkylendiamine ausgewählt.

Unter Alkylendiaminen sind Verbindungen der allgemeinen Formel R⁴R⁵N-Z-NR⁶R⁷ zu verstehen bei denen R⁴, R⁵, R⁶ und R⁷ unabhängig voneinander H, Alkyl- oder Cycloalkylreste sein können. Z bedeutet eine lineare oder verzweigte gesättigte oder ungesättigte Alkylenkette mit zwei, vorzugsweise mehr als zwei C-Atomen. Bevorzugte Beispiele sind Diaminoethan, Diaminopropan, 1,2-Diamino-2-methylpropan, 1,3-Diamino-2,2-dimethylpropan, Diaminobutan, Diaminopentan, 1,5-Diamino-2-methylpentan, Neopentyldiamin, Diaminohexan, 1,6-Diamino-2,2,4-trimethylhexan, 1,6-Diamino-2,4,4-trimethylhexan, Diaminoheptan, Diaminooctan, Diaminononan, Diaminodecan, Diaminoundecan. Diaminododecan, Dimeramin (kommerziell beispielsweise unter dem Handelsnamen Versamin 551 von der Fa. Cognis bzw. BASF erhältlich), Triacetondiamin, Dioxadecandiamin, N,N-Bis(3-Aminopropyl)- dodecylamin (kommerziell beispielsweise unter dem Handelsnamen Lonzabac 12.30 von der Fa. Lonza erhältlich) oder Gemische daraus.

Unter Cycloalkylendiaminen sind Verbindungen der allgemeinen Formel R⁸R⁹N-Y-NR¹⁰R¹¹ zu verstehen bei denen R⁸, R⁹, R¹⁰ und R¹¹ unabhängig voneinander H, Alkyl- oder Cycloalkyreste sein können. Y bedeutet einen gesättigten oder ungesättigten Cycloalkylrest mit mehr als drei C-Atomen, vorzugsweise mehr als vier C-Atomen. Bevorzugt sind Diaminocyclopentane, Diaminocyclohexane, Diaminocycloheptane, beispielsweise 1,4-Cyclohexandiamin, 4,4'-Methylenbiscyclohexylamin, 4,4'-Isopropylen-biscyclohexylamin, Isophorondiamin, m-Xylylendiamin, N-Aminoethylpiperazin oder Gemische daraus.

Die Diamine können auch sowohl Alkyl- als auch Cycloalkylreste gemeinsam enthalten. Bevorzugte Beispiele sind Aminoethylpiperazin, 1,8-Diamino-p-menthan, Isophorondiamin, 1,2-(Bisaminomethyl)-cyclohexan, 1,3-(Bisaminomethyl)-cyclohexan, 1,4-(Bisaminomethyl)-cyclohexan und Bis-(4-aminocyclohexyl)-methan.

Weitere Beispiele für als Komponente (B1) erfindungsgemäss einsetzbare Diamine sind Bis-(6-aminohexyl)-amin, α,α'-Diaminoxylole, usw.

Bevorzugt werden als Komponente (B1) und/oder Komponente (B2) polyfunktionelle Amine eingesetzt. Insbesondere sind dies amin-funktionalisierte Polyalkylenglykole, wie 1,2-Bis-(aminoethoxy)-ethan, 1,13-Diamino-4,7,10-trioxatridecan. Erfindungsgemäß einsetzbare amin-funktionalisierte Polyalkylenglykole sind kommerziell beispielsweise als Jeffamine(R) über die Firma Huntsman Corp. erhältlich. Bevorzugt sind Jeffamine: D-230, D-400, D-2000, D-4000, T-403, T-3000, T-5000, ED-600, ED-2003.

Ebenfalls bevorzugt als Komponente (B1) und/oder Komponente (B2) einsetzbare polyfunktionelle Amine sind Verbindungen der allgemeinen Formel H₂N-(CH₂CH₂-NH)ₓ-CH₂CH₂-NH₂, mit 1<x<10, wie z.B. Diethylentriamin, Triethylentetramin, Tetraethylenpentamin, Pentaethylenhexamin, Dipropylentriamin, Bis-(3-aminopropyl)-amin, N,N-Bis(3-aminopropyl)-ethylendiamin, Bishexamethylentriamin, Heptaethylenoctamin und ähnliche.

Als Komponente (B2) werden bevorzugt Polymere eingesetzt, die ausgewählt sind aus der Gruppe der Polyamine, Polyimine, Polyether, Polyamide, Polyaminoamide, Polyurethane, Polyolefine, Polyvinylamine oder Mischungen daraus.

Polyamine, die als Komponente (B2) einsetzbar sind, werden bei Henry Lee und Kris Neville, Handbook of Epoxy Resins, Kapitel 7, Seiten 7-1 bis 7-33, McGrawHill Book Company, New York 1967 und dort zitierte Literatur, sowie bei Clayton A. May, Epoxy Resins, Seiten 466 - 468, Marcel Dekker, New York 1988 und dort zitierte Literatur, beschrieben.

Bevorzugte Polyimine sind Polyethylenimine. Die Aminwasserstofffunktionen der Polyethylenimine können auch teilweise modifiziert sein, wie zum Beispiel durch Alkylierung, Benzylierung, Acylierung, Alkoxylierung, vorzugsweise Ethoxylierung und/oder Propoxylierung. Insbesondere bevorzugt ist die Modifizierung mit Epichlorhydrin. Bevorzugt einsetzbare Polyethylenimine sind kommerziell von der Firma BASF unter dem Handelsnamen Lupasol(R) PS, P, WF, Bo 150, FC, FG, G100, G-20, G-35, G-500, HF, PO-100, PR-8515 und SK oder von der Firma DOW unter dem Handelsnamen Polyethylenimin 6, 12, 18, 600, 1000 erhältlich.

Polyaminoamide enthalten in der Hauptkette sowohl Amin- als auch Amid-Funktionalitäten. Polyaminoamide werden durch Polykondensation von Polyaminen und Dicarbonsäuren oder durch Michael-Addition von Acrylsäureestern an Diamine und anschließender Polykondensation der resultierenden Aminosäureester hergestellt. Polyaminoamide, die als Komponente (B2) einsetzbar sind, werden bei Henry Lee und Kris Neville, Handbook of Epoxy Resins, Kapitel 10, Seiten 10-1 bis 10-23, McGraw-Hill Book Company, New York 1967, sowie bei Clayton A. May, Epoxy Resins, Seiten 469, Marcel Dekker, New York 1988 und dort zitierte Literatur, beschrieben.

Im Rahmen der vorliegenden Erfindung werden bevorzugt Polyaminoamide eingesetzt, die erhältlich sind durch Polykondensation von aliphatischen Polyaminen und dimerisierten oder trimerisierten Fettsäuren. Einsetzbar sind auch die ungepfropften und gepfropften Polyaminoamide, wie sie in der WO 94/29422 beschrieben sind. Kommerziell sind Polyaminoamide von der Firma Cognis bzw. BASF unter dem Handelsnamen Versamid®, von der Firma Bakelite AG unter dem Handelsnamen Ruetadur oder der Firma S. I. Q. Kunstharz GmbH aus der SIQTherm-Produktreihe erhältlich.

Weitere bevorzugt als Komponente (B2) einsetzbare Polyamine sind Polyvinylamine. Polyvinylamine können beispielsweise hergestellt werden durch Polymerisation von N-Vinylacylaminen, wie N-Vinylformamid, N-Vinylacetamid, usw. und anschließender vollständiger oder teilweiser Hydrolyse der Amidgruppe. Bevorzugt einsetzbare Polyvinylamine sind kommerziell von der Firma BASF unter dem Handelsnamen Lupamin®: 1500, 4500, 4595, 9000, 9030, 9095 erhältlich. Aminterminierte Polyetherurethane sind beispielsweise von der Firma Henkel unter dem Handelsnamen Loctite Liofol UR 9640 erhältlich.

Weitere, als Komponente (B2) einsetzbare Polyamine sind hochverzweigte Polymere, die an den Zweigenden Aminogruppen, insbesondere primäre Aminogruppen, tragen.

Eine als Komponente (B2) besonders bevorzugte Gruppe hochverzweigter Polymerer sind die dendritischen Polymeren, die auch als Dendrimere, Kaskadenpolymere oder "starburst"-Polymere bezeichnet werden. Hierunter werden synthetische Makromoleküle verstanden, die man schrittweise durch Verknüpfung von jeweils 2 oder mehr Monomeren mit jedem bereits gebundenen Monomeren aufbaut, so dass mit jedem Schritt die Zahl der Monomer-Endgruppen exponentiell anwächst und am Ende eine kugelförmige Baumstruktur entsteht. Bevorzugte Dendrimere sind Polyaminoamide (PAMAM) mit primären Aminofunktionen an den Zweigenden, bevorzugt sind solche der Generation > 0. Unter der Generation 0 werden Dendrimere mit folgender Struktur verstanden:

[-CH₂N(CH₂CH₂CONHCH₂CH₂NH₂)₂]₂,

Besonders bevorzugt sind Dendrimere der Generation >1 , wobei Dendrimere der Generation 1 folgende Struktur aufweisen:

[-CH₂N[CH₂CH₂CONHCH₂CH₂N(CH₂CH₂CONHCH₂CH₂NH₂)₂]₂]₂

Die Strukturen der höheren Generationen, bevorzugt bis Generation 6, ergeben sich aus dem obigen systematischen Aufbau von Generation 0 zur Generation 1.

Dendrimere können hergestellt werden zum Beispiel durch Stufenreaktion von Ammoniak oder von geeigneten Vertretern der oben erwähnten Alkylendiamine der allgemeinen Formel R⁴R⁵N-Z-NR⁶R⁷ mit Acrylsäureestem. R⁴-R⁷ bedeuten in diesen Fällen Wasserstoff. Z bedeutet eine lineare oder verzweigte gesättigte oder ungesättigte Alkylenkette mit zwei oder mehr C-Atomen. Der Polymeraufbau findet dabei statt durch Michael-Addition der Aminogruppen an die olefinischen Doppelbindungen und Kondensation von Aminogruppen mit Estergruppen. Es ist dabei ein geeigneter molarer Überschuss an Aminen zu wählen. Weitere geeignete Aminkomponenten für den Dendrimeraufbau finden sich in den oben erwähnten Gruppen der Cycloalkylendiamine, der Diamine die sowohl Alkyl- als auch Cycloalkylreste besitzen und der Gruppe der aminfunktionalisierten Polyalkylenglykole. Alle in Frage kommenden Aminbausteine besitzen in diesen Fällen 2 primäre Aminofunktionen.

Eine weitere bevorzugte Gruppe hochverzweigter Polymerer, die als Komponente (B2) eingesetzt werden, entsteht zum Beispiel durch Stufenreaktion von Acrylsäureestem mit geeigneten Vertretern der oben erwähnten polyfunktionellen Amine der allgemeinen Formel H₂N-(CH₂CH₂-NH)X-CH₂CH₂-NH₂ mit 1<x<10, wie z.B. Diethylentriamin, Triethylentetramin, Tetraethylenpentamin und Pentaethylenhexamin.

Die erfindungsgemäß einsetzbare Komponente (B2) kann auch durch Reaktion eines Überschusses der oben erwähnten, als Komponente (B1) einsetzbaren niedermolekularen polyfunktionellen Amine mit cyclischen Carbonaten hergestellt werden, die ein mittleres Molekulargewicht (Mw) von weniger als 1.000 g/mol, bevorzugt von 100 g/mol bis 800 g/mol bis aufweisen. In diesen Fällen ist ein geeigneter molarer Überschuss an Amin in Relation zu Cyclocarbonat zu wählen, so dass einerseits das gewünschte Molekulargewicht erreicht und zusätzlich die erfindungsgemäße Aminfunktionalität für den Einsatz als Komponente (B2) vorhanden ist.

Die Komponente (B2) wird als Einzelkomponente oder auch als Mischung der entsprechenden, als Komponente (B2) einsetzbaren Verbindungen eingesetzt.

Das hierin beschriebene Bindemittel-System eignet sich insbesondere als Kleb/Dichtstoff.

Gegenstand der vorliegenden Erfindung ist daher auch ein Verfahren zur Herstellung eines Kleb-/Dichtstoffes unter Verwendung des hierin beschriebenen Bindemittel-Systems, wobei man Komponente (A) mit Komponente (B) im Verhältnis der Carbonatgruppen zu primären Aminogruppen von 30:1 bis 0,2:1, vorzugsweise 10:1 bis 0,4:1, bevorzugter von 5:1 bis 0,5:1, besonders bevorzugt von 2:1 bis 0,6:1 und am meisten bevorzugt ungefähr 1:1 mischt. Sind keine primären Aminogruppen im Molekül enthalten, so ist das Verhältnis auf die sekundären Aminogruppen anzuwenden. Dabei sind jeweils die funktionellen Gruppen der Komponenten A sowie B1, B2 insgesamt zu berücksichtigen.

In einer bevorzugten Ausführungsform des hierin beschriebenen Verfahrens geschieht die Reaktion zwischen Komponente (A) und Komponente (B) in Gegenwart eines Lösemittels.

Als Lösemittel sind grundsätzlich alle dem Fachmann bekannte Lösemittel verwendbar, insbesondere Ketone, halogenierte Kohlenwasserstoffe, Alkane, Alkene und aromatische Kohlenwasserstoffe. Beispiele für solche Lösemittel sind Methylenchlorid, Trichlorethylen, Toluol, Xylol, Butylacetat, Amylacetat, Isobutylacetat, Methylisobutylketon, Methoxybutylacetat, Cyclohexan, Cyclohexanon, Dichlorbenzol, Diethylketon, Di-isobutylketon, Dioxan, Ethylacetat, Ethylenglykolmonobutyletheracetat, Ethylenglykolmonoethylacetat, 2-Ethylhexylacetat, Glykoldiacetat, Heptan, Hexan, Isobutylacetat, Isooctan, Isopropylacetat, Methylethylketon, Tetrahydrofuran oder Tetrachlorethylen oder Mischungen aus zwei oder mehr der genannten Lösemittel.

In einer besonderen Ausführungsform des hierin beschriebenen Verfahrens geschieht die Reaktion zwischen Komponente (A) und Komponente (B) unter Katalyse. Hierzu werden der Mischung katalytische Mengen einer Base hinzugegeben. Derartige Basen und einzusetzende Mengen sind in der US 5,977,266 und der WO 02/079148 beschrieben. Insbesondere wird auf die WO 98/50345, Seite 3, Zeile 1 bis Seite 4, Zeile 17, Bezug genommen. Der Katalysator kann aber auch bereits in Komponente (A) oder (B) enthalten sein. Zur Verklebung oder Abdichtung wird mindestens eine Seite eines zu verklebenden oder zu dichtenden Substrats mit der Mischung beschichtet und die so beschichtete Seite mit mindestens einem weiteren Substrat verbunden.

Das hierin beschriebene Bindemittel-System eignet sich zum Verkleben und Dichten der unterschiedlichsten Substrate. Zu diesen Substraten zählen beispielsweise Holz, Metall, Glas, Pflanzenfasern, Stein, Papier, Cellulosehydrat, Kunststoffe wie Polystyrol, Polyethylen, Polypropylen, Polyethylenterephthalat, Polyvinylchlorid, Copolymere von Vinylchlorid und Vinylidenchlorid, Copolymere von Vinylacetatolefinen, Polyamide, insbesondere Kunststofffolien, Metalle, insbesondere Folien aus Aluminium, Blei oder Kupfer.

Insbesondere eignet sich das hierin beschriebene Bindemittel-System als Zweikomponenten-Klebstoff zum Verkleben von Papier, Pappe, Holz, Kunststoff, Metall oder Steingut.

In einer besonders bevorzugten Ausführungsform der Erfindung wird das hierin beschriebene Bindemittel-System als lösemittelfreier oder lösemittelhaltiger Kaschierklebstoff verwendet. Vorzugsweise ist der Kaschierklebstoff im Wesentlichen lösemittelfrei, insbesondere im Wesentlichen frei von Wasser.

Das hierin beschriebene Bindemittel-System kann mit allen gängigen Auftragsverfahren auf die zu verklebenden Substrate aufgebracht werden, beispielsweise durch Sprühen, Rakeln, 3-4-Walzenauftragswerke im Falle der Anwendung eines lösemittelfreien Bindemittel-Systems oder 2-Walzenauftragswerke im Falle der Anwendung eines lösemittelhaltigen Bindemittel-Systems.

Durch seine niedrige Viskosität eignet sich das hierin beschriebene Bindemittel-System insbesondere zum Verkleben temperaturempfindlicher Kunststofffolien, beispielsweise von Polyolefinfolien, insbesondere von Polyolefinfolien aus Polyethylen oder Polypropylen.

Ein weiterer Gegenstand der vorliegenden Erfindung ist daher auch ein Verfahren zur Herstellung von Folienverbunden, die durch das teil- oder vollflächige Verkleben von mindestens zwei gleichen oder unterschiedlichen Kunststofffolien erhältlich sind, unter Verwendung des hierin beschriebenen Bindemittel-Systems. Der Auftrag des Bindemittel-Systems als Zwei-Komponenten-Klebstoff auf die zu verklebenden Folien kann mit üblicherweise für solche Zwecke eingesetzten Maschinen, beispielsweise mit herkömmlichen Laminiermaschinen, erfolgen. Ein weiterer Gegenstand der Erfindung ist eine Verbundfolie, hergestellt nach dem hierin beschriebenen Verfahren unter Verwendung des hierin beschriebenen Bindemittel-Systems. Die Verbundfolie ist insbesondere zur Verpackung von Lebens- und Genussmitteln und Arzneimitteln geeignet.

Das hierin beschriebene Bindemittel-System kann übliche Additive, wie Weichmacher, Silane, Antioxidantien, UV-Stabilisierungsmittel und Alterungsschutzmittel enthalten. Bevorzugt einsetzbare Weichmacher sind Phthalsäureester, beispielsweise Dioctylphthalat, Ditridecylphthalat und Butylbenzylphthalat, Phosphorsäureester, beispielsweise Tricresylphosphat, Adipate, beispielsweise Dioctyladipat, oder Benzoate, beispielsweise Propylenglykoldibenzoat.

Amino-, Epoxy-, oder Mercaptosilane, insbesondere γ-Glycidyloxypropyl- oder γ-Aminopropyltrimethoxysilan, dienen vor allem der Verbesserung der Haftung auf Glas, Metallen etc.

Zur Verwendung als Dichtungsmasse können dem hierin beschriebenen Bindemittel-System anorganische Füllstoffe wie Ruß, Calciumcarbonat, Titandioxid und dergleichen zugesetzt werden. Als anorganische Füllstoffe finden vorzugsweise hochdisperse Kieselsäuren, vor allem pyrogene Kieselsäuren oder Fällungskieselsäuren Verwendung, welche thixotropierend wirken und deren thixotropierende Eigenschaften in den hierin beschriebenen Bindemittel-Systemen auch nach längerer Lagerung erhalten bleiben.

Zur Verwendung als Kaschierklebstoff ist das Bindemittel-System vorzugsweise frei von anorganischen Füllstoffen.

Nachfolgend soll die Erfindung an einigen exemplarischen Beispielen näher erläutert werden. Dabei sind die angegeben Mengen Gewichtsprozent, falls nicht anders angegeben.

Alle hierin im Zusammenhang mit dem Bindemittel-System offenbarten Ausführungsformen sind natürlich auch auf die beschriebenen Verwendungen und Verfahren anwendbar und umgekehrt.

### Beispiele

### Rohstoffe:

- PPG2000: Voranol 2000L, Propylenglykol, Mw = 2.000 g/mol, Fa. Dow
- Lupranat MIS: Diphenylmethandiisocyanat, Isomerengemisch, Fa. BASF
- Glycerincarbonat: Jeffsol Glycerincarbonat (4-Hydroxymethyl-1,3-dioxolan-2-on), Fa. Huntsman
- TIB Kat 216: Dioctylzinndilaurat, Fa. TIB Chemicals
- Epoxid 1: kommerziell erhältliches, difunktionelles Propylenglykol-basiertes Epoxidharz (EEW 320 g/mol)
- Amin 1: kommerziell erhältliches, difunktionelles Amin mit ausschließlich primären Aminogruppen, Mw = 176 g/mol
- Amin 2: kommerziell erhältliches, hyperverzweigtes Polyethylenimin (Verhältnis primäre/sekundäre/tertiäre Amine: 1/0,9/0,5), Mw = 800 g/mol

### Herstellung der Komponenten:

### Synthese des lösungsmittelfreien Präpolymeren 1 (KA1)

PPG2000 (435,46 g) wird in einem Dreihalskolben vorgelegt, entwässert (10 mbar, 80 °C) für eine Stunde und mit Stickstoff belüftet. Nach dem Abkühlen und über Nacht Lagerung unter Schutzgas wurde Lupranat MIS (104,69 g) zugegeben und nach dem kompletten Lösen wird die Reaktionsmischung wieder auf 80 °C eingestellt. NCO-Titrationen werden regelmäßig durchgeführt und bei NCO = 2,91 % wird Glycerincarbonat (46,30 g) bei niedrigerer Temperatur (50,6 °C) zugegeben. Gleich im Anschluss werden 0,05 Gew.-% TIB Kat 216 (0,30 g) zusätzlich zugegeben und nachdem die Exothermie vorbei ist, wird das Reaktionsgemisch wieder auf 80 °C erhitzt bis NCO < 0,1 %.

### Synthese des lösungsmittelhaltigen Präpolymeren 2 (KA2)

PPG2000 (1548,14 g) wird in einem Dreihalskolben vorgelegt, entwässert (<10 mbar, 80 °C) für eine Stunde und mit Stickstoff belüftet. Nach dem Abkühlen und über Nacht Lagerung unter Schutzgas wurde die Reaktionsmischung auf etwa 35°C eingestellt und Lupranat MIS (371,92 g) zugegeben. Nach dem kompletten Homogenisieren wird die Reaktionsmischung wieder auf 80°C eingestellt. NCO-Titrationen werden regelmäßig durchgeführt und bei NCO = 3,01 % wird Glycerincarbonat (164,69 g) bei niedrigerer Temperatur (50,2 °C) zugegeben. Gleich im Anschluss werden 0,05 Gew.-% TIB Kat 216 (0,30 g) zusätzlich zugegeben und nachdem die Exothermie vorbei ist, wird das Reaktionsgemisch wieder auf 80 °C erhitzt bis NCO < 0,1 %. Dann wird bei ca. 55 °C Ethanol (1390,46 g) zugegeben, so dass ein Feststoffanteil von 60 Gew.-% erreicht wird.

### Komponente A mit Epoxid (KA3)

KA1 (2100 g) wurde in einem Gefäß vorgelegt und Epoxidharz Epoxid 1 (176 g) zugegeben. Die Mischung wurde mit einem Holzspatel bis zur Homogenisierung gerührt und bis zur weiteren Verwendung aufbewahrt.

### Herstellung des Härters (KB1)

Amin 1 (20,00 g) und Amin 2 (80,09 g) wurden bei Raumtemperatur mit einem Magnetrührer gemischt bis die Mischung homogen war.

### Herstellung der Klebstoffe und Kaschierungen:

### Herstellung des lösungsmittelhaltigen Klebstoffs 1 und Laminierung (KS1) (Vergleichsbeispiel)

KA1 (11,00 g) wurde in Ethylacetat (35,4805 g) gelöst. Zusätzlich wurde KB1 (0,8268 g) zugegeben (Verhältnis primäres Amin/Cyclocarbonat = 1/1 - Äquivalent). So entsteht ein Klebstoff mit 25 Gew.-% Feststoffgehalt, der laminiert werden kann (KS1). KS1 wurde mit einer Spiralrakel (0,08 mm) auf eine PET-Folie aufgetragen und das Lösungsmittel 5 Minuten im Trockenschrank bei 90°C abgedampft. Sofort danach wurde die Laminierung mit einer PE Folie (Oberfläche Corona-vorbehandelt) mit Hilfe eines Labor-Laminiergerätes vervollständigt. Das Laminat (2 bis 3,5 g/m², trocken) wurde bei 40°C gelagert unter Druck (5 kg Gewicht) und regelmäßige Streifen zur Bestimmung der Verbundhaftung abgeschnitten (15 mm breite Streifen, 90° Schälversuche an einer Zugprüfmaschine 100 mm/min). Von der PE-Lage des Laminats wurde ein IR-Spektrum aufgenommen (ATR). Verbundhaftung nach 1 Tag bei 40 °C: PET/PE: 0,72 N/15mm, kohäsiver Bruch, beide Streifen noch klebrig. Das IR Spektrum nach 3 Tagen bei 40°C zeigt noch die Bande der Cyclocarbonatcarbonylgruppe bei 1818,30 cm⁻¹.

### Herstellung des lösungsmittelhaltigen Klebstoffs 2 und Laminierung (KS2) (erfindungsgemäß)

KA3 (2276g) wurde in Ethanol gelöst (2330 g) und dazu der Härter KB1 (157 g) gegeben (Verhältnis primäres Amin/Cyclocarbonat = 1/1 - Äquivalent) und mit einem Holzspatel homogenisiert. So entsteht ein Klebstoff mit 32 Gew.-% Feststoffgehalt, der laminiert werden kann (KS2). KS2 wurde mittels einer Labocombi Kaschieranlage mit Walzenauftrag (Gravur Walze mit 50 Linien/cm) aufgetragen und die Parameter so eingestellt, dass das Auftragungsgewicht (trocken) ca. 2,5 g/m² beträgt. Die kaschierte gewickelte Rolle wurde direkt nach der Laminierung bei 40°C gelagert. Verbundhaftung nach 1 Tag bei 40°C: PET/PE: 2,05 N/15mm, adhäsiver Bruch, PE noch klebrig. IR Spektrum nach 2 Tagen bei 40°C zeigt keine Bande der Cyclocarbonatcarbonylgruppe bei 1818,30 cm⁻¹ mehr.

## Patentansprüche

1. Bindemittel-System enthaltend die Komponenten (A) und (B), **dadurch gekennzeichnet, dass**
(i) Komponente (A) mindestens eine mindestens zwei zyklische Carbonatgruppen tragende Verbindung und mindestens eine mindestens zwei Epoxidgruppen tragende Verbindung umfasst; und
(ii) Komponente (B) mindestens eine mindestens zwei (-NHR-)-Atomgruppierungen tragende Verbindung umfasst, wobei R H, ein Alkyl oder Aryl-Rest ist.

2. Bindemittel-System nach Anspruch 1, **dadurch gekennzeichnet, dass** die mindestens eine mindestens zwei zyklische Carbonatgruppen tragende Verbindung ein zyklisches Carbonatgruppen tragendes Polymer ist, ausgewählt aus der Gruppe der fettchemischen Verbindungen, Polyether, Polyetherpolyole, Polyester, Polyesterpolyole, Polycarbonate, Polycarbonsäuren, Polyacrylate, Polymethacrylate, Polyamide, Polyamine, Polyurethane, oder Mischungen daraus.

3. Bindemittel-System nach Anspruch 1 oder 2, **dadurch gekennzeichnet, dass** die mindestens eine mindestens zwei zyklische Carbonatgruppen tragende Verbindung ein Umsetzungsprodukt ist von zyklischem Hydroxyalkylcarbonat mit einem Isocyanatgruppen-tragenden Polymer.

4. Bindemittel-System nach Anspruch 3, **dadurch gekennzeichnet, dass** das zyklische Hydroxyalkylcarbonat einen 5-gliedrigen oder 6-gliedrigen Carbonatring aufweist, insbesondere Glycerincarbonat ist.

5. Bindemittel-System nach Anspruch 3 oder 4, **dadurch gekennzeichnet, dass** das Isocyanatgruppen-tragende Polymer ein Isocyanatgruppen-terminiertes Polyurethanpräpolymer ist, das durch die Umsetzung von einem Polyol, insbesondere einem Polyol mit einem mittleren gewichtsmittlerem Molekulargewicht Mw von 60 bis 4.000 g/mol, vorzugsweise 75 bis 2.000 g/mol (gemessen mittels Gelpermeationschromatographie (GPC) gemäß der Norm DIN 55672-1:2007-08), mit einem aromatischen Diisocyanat, insbesondere einem Methylendiphenyldiisocyanat, erhältlich ist.

6. Bindemittelsystem nach einem der Ansprüche 1 bis 5, **dadurch gekennzeichnet, dass** die mindestens zwei Epoxidgruppen tragende Verbindung ein aliphatisches Epoxid, insbesondere ein aliphatisches Epoxid mit einem EEW von 100 bis 500 g/mol, vorzugsweise von 120 bis 350 g/mol, ist.

7. Bindemittel-System nach einem der Ansprüche 1 bis 6, **dadurch gekennzeichnet, dass** die mindestens eine mindestens zwei (-NHR-)-Atomgruppierungen tragende Verbindung
(i) ausgewählt ist aus der Gruppe der Alkylendiamine, Cycloalkylendiamine, aminfunktionalisierten Polyalkylenglykole und/oder polyfunktionellen Amine; oder
(ii) ein Polymer ist, insbesondere ein hoch verzweigtes Polymer, vorzugsweise ein Dendrimer, ausgewählt aus der Gruppe der Polyamine, Polyimine, Polyether, Polyamide, Polyaminoamide, Polyurethane, Polyolefine, Polyvinylamine oder Mischungen daraus;
(iii) oder eine Mischung aus den Verbindungen gemäß (i) und (ii) ist.

8. Verfahren zur Herstellung eines Kleb-/Dichtstoffes unter Verwendung des Bindemittel-Systems nach einem der Ansprüche 1 bis 7, **dadurch gekennzeichnet, dass** man Komponente (A) mit Komponente (B) im Verhältnis der Carbonatgruppen zu primären Aminogruppen von 30:1 bis 0,2:1, bevorzugt 10:1 bis 0,4:1, weiter bevorzugt von 5:1 bis 0,5:1, besonders bevorzugt von 2:1 bis 0,6:1, insbesondere bevorzugt 1,1:1 bis 0,9:1 und am meisten bevorzugt ungefähr 1:1 mischt, wobei in Abwesenheit von primären Aminogruppen das Mischungsverhältnis auf sekundäre Aminogruppen anzuwenden ist.

9. Verwendung des Bindemittel-Systems nach einem der Ansprüche 1 bis 7 als Zweikomponenten-Klebstoff zum Verkleben von Papier, Pappe, Holz, Kunststoff, Metall oder Steingut.

10. Verwendung des Bindemittel-Systems nach Anspruch 9 als lösemittelfreier oder lösemittelhaltiger Kaschierklebstoff oder zur Herstellung von Vergussmassen.

11. Verfahren zur Herstellung von Folienverbunden, **dadurch gekennzeichnet, dass** man mindestens zwei gleiche oder unterschiedliche Kunststofffolien teil- oder vollflächig verklebt unter Verwendung eines Bindemittel-Systems nach einem der Ansprüche 1 bis 7.

12. Folienverbund, hergestellt nach dem Verfahren gemäß Anspruch 11.

## Claims

1. A binder system containing components (A) and (B), **characterized in that**
(i) component (A) comprises at least one compound carrying at least two cyclic carbonate groups and at least one compound carrying at least two epoxide groups; and
(ii) component (B) comprises at least one compound carrying at least two (-NHR-) atomic groups, where R is H, an alkyl or aryl functional group.

2. The binder system according to claim 1, **characterized in that** the at least one compound carrying at least two cyclic carbonate groups is a polymer carrying cyclic carbonate groups, selected from the group of fatty chemical compounds, polyethers, polyether polyols, polyesters, polyester polyols, polycarbonates, polycarboxylic acids, polyacrylates, polymethacrylates, polyamides, polyamines, polyurethanes, or mixtures thereof.

3. The binder system according to claim 1 or 2, **characterized in that** the at least one compound carrying at least two cyclic carbonate groups is a reaction product of cyclic hydroxyalkyl carbonate with an isocyanate-group-carrying polymer.

4. The binder system according to claim 3, **characterized in that** the cyclic hydroxyalkyl carbonate has a 5-membered or 6-membered carbonate ring, in particular glycerol carbonate.

5. The binder system according to claim 3 or 4, **characterized in that** the isocyanate-group-carrying polymer is an isocyanate-group-terminated polyurethane prepolymer obtained by the reaction of a polyol, in particular a polyol having an average weight-average molecular weight Mw of 60 to 4,000 g/mol, preferably 75 to 2,000 g/mol (measured by gel permeation chromatography (GPC) according to the DIN 55672-1: 2007-08 standard), with an aromatic diisocyanate, in particular a methylene diphenyl diisocyanate.

6. The binder system according to one of claims 1 to 5, **characterized in that** the compound carrying at least two epoxide groups is an aliphatic epoxide, in particular an aliphatic epoxide having an EEW of 100 to 500 g/mol, preferably 120 to 350 g/mol.

7. The binder system according to one of claims 1 to 6, **characterized in that** the at least one compound carrying at least two (-NHR-) atomic groups
(i) is selected from the group of alkylenediamines, cycloalkylenediamines, amine-functionalized polyalkylene glycols and/or polyfunctional amines; or
(ii) is a polymer, in particular a highly branched polymer, preferably a dendrimer, selected from the group of polyamines, polyimines, polyethers, polyamides, polyaminoamides, polyurethanes, polyolefins, polyvinylamines or mixtures thereof;
(iii) or is a mixture of the compounds according to (i) and (ii).

8. A method for preparing an adhesive/sealant using the binder system according to one of claims 1 to 7, **characterized in that** component (A) is mixed with component (B) with a ratio of carbonate groups to primary amino groups of from 30:1 to 0.2:1, preferably from 10:1 to 0.4:1, more preferably from 5:1 to 0.5:1, particularly preferably from 2:1 to 0.6:1, more particularly preferably from 1.1:1 to 0.9:1, and most preferably approximately 1:1, the mixing ratio being applied to secondary amino groups in the absence of primary amino groups.

9. The use of the binder system according to one of claims 1 to 7 as a two-component adhesive for bonding paper, cardboard, wood, plastic, metal or earthenware.

10. The use of the binder system according to claim 9 as a solvent-free or solvent-containing laminating adhesive or for preparing casting compounds.

11. A method for producing film composites, **characterized in that** at least two identical or different plastics films are bonded over part or all of the surfaces thereof using a binder system according to one of claims 1 to 7.

12. A film composite, produced using the method according to claim 11.

## Revendications

1. Système de liant comprenant les composants (A) et (B), **caractérisé en ce que**
(i) le composant (A) comprend au moins un composé portant au moins deux groupes carbonate cycliques et au moins un composé portant au moins deux groupes époxyde ; et
(ii) le composant (B) comprend un groupe portant au moins un groupement d'atomes (-NHR-), R étant H, un alkyle ou un radical aryle.

2. Système de liant selon la revendication 1, **caractérisé en ce que** l'au moins une liaison portant au moins deux groupes carbonate cycliques est un polymère cyclique portant des groupes carbonate, choisi dans le groupe des composés chimiques gras, des polyéthers, des polyétherpolyols, des polyesters, des polyesterpolyols, des polycarbonates, des acides polycarboxyliques, des polyacrylates, des polyméthacrylates, des polyamides, des polyamines, des polyuréthanes ou de mélanges de ceux-ci.

3. Système de liant selon la revendication 1 ou 2, **caractérisé en ce que** l'au moins un composé portant au moins deux groupes carbonate cycliques est un produit de réaction d'hydroxyalkylcarbonate cyclique avec un polymère portant un groupe isocyanate.

4. Système de liant selon la revendication 3, **caractérisé en ce que** le carbonate d'hydroxyalkyle cyclique présente un cycle carbonate à 5 ou 6 membres, en particulier le carbonate de glycérol.

5. Système de liant selon la revendication 3 ou 4, **caractérisé en ce que** le polymère portant un groupe isocyanate est un prépolymère de polyuréthane terminé par des groupes isocyanate, lequel peut être obtenu par la réaction d'un polyol, en particulier d'un polyol ayant un poids moléculaire moyen en poids Mw de 60 à 4 000 g/mol, de préférence de 75 à 2 000 g/mol (mesuré par chromatographie par perméation de gel (CPG) selon la norme DIN 55672-1:2007-08), avec un diisocyanate aromatique, en particulier un méthylènediphényldiisocyanate.

6. Système de liant selon l'une quelconque des revendications 1 à 5, **caractérisé en ce que** le composé portant au moins deux groupes époxyde est un époxyde aliphatique, en particulier un époxyde aliphatique ayant un EEW de 100 à 500 g/mol, de préférence de 120 à 350 g/mol.

7. Système de liant selon l'une quelconque des revendications 1 à 6, **caractérisé en ce que** l'au moins un composé portant au moins deux groupements d'atomes (-NHR-)
(i) est choisi dans le groupe des alkylènediamines, des cycloalkylènediamines, des polyalkylèneglycols à fonctionnalité amine et/ou des amines polyfonctionnelles ; ou
(ii) est un polymère, en particulier un polymère hautement ramifié, de préférence un dendrimère, choisi dans le groupe des polyamines, des polyimines, des polyéthers, des polyamides, des polyaminoamides, des polyuréthannes, des polyoléfines, des polyvinylamines ou de mélanges de ceux-ci ;
(iii) ou un mélange des composés selon (i) et (ii).

8. Procédé pour la préparation d'un agent adhésif/d'étanchéité à l'aide du système de liant selon l'une quelconque des revendications 1 à 7, **caractérisé en ce qu'**on mélange le composant (A) avec le composant (B) dans le rapport entre les groupes carbonate et les groupes amino primaires de 30:1 à 0,2:1, de préférence 10:1 à 0,4:1, de manière davantage préférée de 5:1 à 0,5:1, de manière particulièrement préférée de 2:1 à 0,6:1, de manière particulièrement préférée 1,1:1 à 0,9:1, et de manière préférée entre toutes d'environ 1:1, le rapport de mélange devant être appliqué aux groupes amino secondaires en l'absence de groupes amino primaires.

9. Utilisation du système de liant selon l'une quelconque des revendications 1 à 7 en tant qu'adhésif à deux composants pour le collage de papier, de carton, de bois, de plastique, de métal ou de faïence.

10. Utilisation du système de liant selon la revendication 9 comme un adhésif de stratification, sans solvant ou contenant un solvant ou pour la production de masses de scellement.

11. Procédé de fabrication de feuilles complexes, **caractérisé en ce que** l'on colle au moins deux films plastiques identiques ou différents, partiellement ou totalement liés, à l'aide d'un système de liant selon l'une quelconque des revendications 1 à 7.

12. Feuille complexe fabriquée selon le procédé selon la revendication 11.
